# EUROPEAN PATENT APPLICATION

(11) **EP 2 147 719 A1**
(43) Date of publication of application: **27.01.2010**
(21) Application number: 08733969.3
(22) Date of filing: 14.04.2008
(51) Int. Cl.: B01J 23/46, B01J 29/40

(54) **THE CATALYST USED IN THE REACTION OF BROMINATING OXIDATION OF METHANE AND THE CATALYST USED IN THE FARTHER REACTION OF PRODUCING HIGHER HYDROCARBON**

(30) Priority: 13.04.2007 CN 200710034726
(71) Applicant: Microvast Technologies, Ltd., Zhejiang 313000 (CN)
(72) Inventor: LIU, Zhen, Changsha, Hunan 410082 (CN); ZHANG, Hongmin, Changsha, Hunan 410082 (CN); LI, Wensheng, Hunan 410082 (CN); REN, Yanqun, Changsha, Hunan 410082 (CN); ZHOU, Xiaoping, Zhejiang 313000 (CN)
(74) Representative: Tomlinson, Edward James
(86) International application number: PCT/CN2008/000771
(87) International publication number: WO 2008/125017

(57) **Abstract**

A catalyst used in the reaction of oxidative bromination of methane is provided. The catalyst is prepared by the following procedures: mixing at least one of the precursors selected from the compounds of Rh, Ru, Cu, Zn, Ag, Ce, V, W, Cd, Mo, Mn, Cr and La which can dissolve in water with the Si precursor, hydrolyzing, drying and sintering. In the catalysis system, methane reacts with HBr, H₂O and oxygen source (O₂, air or oxygen-rich air), finally CH₃Br and CH₂Br₂ are produced. Another catalyst used in the reaction of condensation of methane bromide to C₃-C₁₃ hydrocarbons is also provided. This catalyst is prepared by supporting compounds of Zn or Mg on molecular sieves such as HZSM-5, HY, Hb, 3A, 4A, 5A or 13X et al. With this catalyst, CH₃Br and CH₂Br₂ produced in the former process can react further to give C₃ to C₁₃ hydrocarbons and HBr, and HBr can be recycled as a medium.

## Description

### FIELD OF THE INVENTION

The present invention relates to catalysts for transforming methane to methane bromides and catalysts for the further conversion of methane bromides to higher hydrocarbons, belonging to the field of catalytic conversion of methane to chemicals.

### BACKGROUND OF THE INVENTION

As the continuous decrease of petroleum resources, the conversion of natural gas to high value chemicals has attracted more attention. In China, the reserve of natural gas is roughly 124 trillion cubic meters. Natural gas is mainly composed of methane from about 60% to about 99%, with a small amount of other compounds such as ethane and propane.etc. Large amounts of lower alkanes from the exploitation of oil fields were flared, not only wasting the natural resources, but also emitting carbon dioxide into atmosphere. So developing effective utilization method of methane plays an importance role in economy.

Methane (CH₄) is a compound with the molecule structure similar to that of inert gases, have strong C-H bonds (average bond energy 414 kJ/mol), making it very difficult to be activated under mild condition. The C-H bond dissociation energies of CH₄, CH₃, CH₂ and CH are 420 kJ/mol, 360 kJ/mol, 520 kJ/mol and 330 kJ/mol, respectively. Studying the activation and selectively conversion of C-H bond could provide reference for the conversion of other hydrocarbons.

The conversion of natural gas into liquid chemicals provides an effective solution for utilization of natural gas from remote areas. The traditional method for the utilization of natural gas is to transform it to Synthesis Gas (Syngas), which is then converted to liquid products. For instance, synfuels were produced from Syngas via Fischer-Tropsch (F-T) process (Fischer, F., and Tropsch, H., Brennst. Chem. 4, 276 (1923)). Although the F-T method offers a process for the conversion of natural gas into more easily transported liquid fuels, the reaction of the production of Syngas is strongly endothermic reaction, and there is a significant energy needed during the reaction.

Periana had made the research on converting methane into methanol (Roy A., Periana et al., Science, 280, 560(1998)) and acetic acid (Roy A. Periana, et al., Science, 301, 814(2003)). In the process, SO₂ was produced, which could not be recovered.And oil of vitriol, used as a reactant and solvent, was diluted in the reaction, and could not be used after a while Periana's group and Catalytica Inc. have made some great efforts in this development, nevertheless the method has not been industrialized.

Recently, a novel process for the activation of methane designed at inventor's group was attracting some attention, which comprises: firstly transforming methane to methyl bromide via oxidative bromination, secondly transforming methyl bromide into chemicals, such as methanol, dimethyl ether, light alkenes and higher olefins including gasoline.

In this field, we have developed processes for the synthesis of acetic acid, dimethyl ether and higher hydrocarbonds from methane (patent applications and pending: CN200410022850.8, CN200510031734.7, CN200610031377.9). These processes comprise: firstly transforming alkanes of natural gas into alkyl bromides and carbon monoxide, and secondly the alkyl bromides were converted into corresponding products, meanwhile recovering HBr. HBr was then introduced into first reactor to produce alkyl bromides to complete its cycle. When the high selectivity of alkyl bromides were obtained in the first step, methanol, dimethyl ether and higher hydrocarbonds could be produced in the second step. When the mixture composed of alkyl bromides and carbon monoxide was produced in the first step, acetic acid could be prepared subsequently. The novelty of the processes lies in the acquisition of differently applicable alkyl bromides and carbon monoxide through adjusting the catalysts used for the first step.

So far, no similar process has been reported. Olah (G. A. Olah, A. Molnar, Hydrocarbon Chemistry (Wiley, New York, 1995)) reported a process to form CH₃Br and HBr by reacting methane and Br₂, then hydrolyzing CH₃Br to provide methanol and dimethyl ether. The reported single-pass conversion of methane is lower than 20%.

Xiao Ping Zhou et al from GRT Inc. had designed a process to convert alkane to methanol and dimethyl ether using Br₂ as media (CN02827498.9, US6472572, US6462243). The difference between this process and Olah's development was that GRT's process included the following steps: alkanes react with Br₂ to give alkyl bromide and HBr, the resulted mixture further reacting with one kind of metal oxide to provide target products and metal bromide, finally metal bromide reacts with O₂ to regenerate Br₂ and metal oxide. However, the process relates to the use of Br₂ and the extra step of regenerating Br₂, as known, the utilization and storage of vast amount of Br₂ is very dangerous. Moreover, as the reaction is a stoichiometric reaction instead of a catalytical one, metal oxide is transferred in the reaction system, thus it results a complicated process and increases energy consumption as well.

Dow Global reported the processes for preparing methanol, dimethyl ethe, light alkene such as ethylene, propylene and butylenes, higher olefins including gasoline, vinyl halide monomer and acetic acid using chlorine or hydrogen chloride as media (CN02813796.5). However, in these processes, chloromethane was synthesized via hydrogen chloride and used as an intermediate. Metal oxyhalides without any support was used as the catalyst, and this could cause the instability of the catalyst. The rare earth metal elements were used in the metal oxyhalides, and it would add a significant cost in the process. Only 13.3% single-pass conversion rate was achieved.

### SUMMARY OF THE INVENTION

The present invention provides further improvements of the catalyst used for the oxidative bromination of the lower hydrocarbons, such as methane (or natural gas), and the catalyst used for the conversion from alkyl bromides into higher hydrocarbons.

### DETAILED DESCRIPTION OF THE INVENTION

One object of the present invention is to provide an optimized catalyst used for the conversion of methane of the natural gas into alkyl bromides. The catalyst was a compound catalyst of metals or metal oxides distributed in silicon dioxide solidoid, prepared by the hydrolyzation, drying, and then calcination of the mixture formed by corresponding dissolvable metal compound precursors and silicon precursor.

In the process of this invention, silicon dioxide was used as support for the first catalyst, and at least one dissolvable chloride, bromide or nitrate of metal selected from the group consisting of Ru, Rh, Pd, Pt, Ni, Cu, Zn, Ag, Ce, V, W, Cd, Mo, Mn, Cr and La was used for the metal compound precursors.

First catalyst was used for first step that has the following reaction formula :

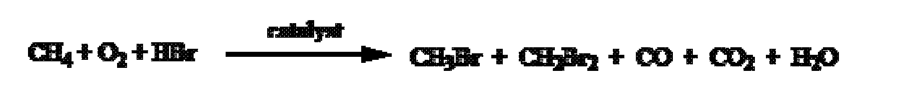

The present invention provides a catalyst for the conversion of methane to alkyl bromides via oxidative bromination. In the process, contacting methane, hydrogen bromide, H₂O and oxygen source (oxygen, air or oxygen-rich air) over the catalyst in a fixed bed reactor, under a certain reaction temperature, a product mixture, comprising CH₃Br, CH₂Br₂ and by-products carbon monoxide and carbon dioxide, will be produced. The method of higher hydrocarbons synthesis from CH₃Br and CH₂Br₂ was given in our application CN200610031377.9.

The oxidative bromination catalyst of the present invention is a compound catalyst of metals or metal oxides distributed in silicon dioxide. In the process of this invention, the preparation of the catalyst comprises, firstly preparing of metal salt solution using at least one chloride, bromide or nitrate of metal selected from the group consisting of Ru, Rh, Pd, Pt, Ni, Cu, Zn, Ag, Ce, V, W, Cd, Mo, Mn, Cr and La, and preparing of silica sol by the hydrolyzing of SiCl₄ or silicon ester or directly using the commercial silica sol. And then mixing the metal salt solution with silica sol and subsequently drying, calcining the mixture to obtain the catalyst.

According to the present invention, among the metal salts of chloride, bromide or nitrate of metal selected from the group consisting of Ru, Rh, Pd, Pt, Ni, Cu, Zn, Ag, Ce, V, W, Cd, Mo, Mn, Cr and La, the chloride, bromide or nitrate of Ru or Rh is preferable. In the catalyst, the mass content of Ru is in the range of 0.10% to 2.0%, preferably 0.40% to 1.40%, and more preferably 0.60% to 1.20%. In the catalyst, the mass ratio of Rh is in the range of 0.10% to 0.80%, preferably 0.20% to 0.50%, and more preferably 0.30% to 0.50%. In the preparation of the catalyst, the temperature of calcination is in the range of 500°C to 1200°C, preferably 700°C to 1000°C, and more preferably 700°C to 900°C.

In the process, the reaction temperature of methane, hydrogen bromide, H₂O and oxygen over the catalyst is in the range of 500°C to 750°C, preferably 600°C to 800°C, and more preferably 620°C to 670°C.

Another object of the present invention is to provide a catalyst used for the alkyl bromides conversion to higher hydrocarbons. The following reaction formula illustrates the bromomethane and dibromomethane conversion to higher hydrocarbons ( >C₃).

The catalyst for this reaction was formed by the metal compound supported on the 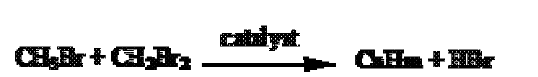 molecular sieve selected from the group of HZSM-5, HY, Hβ, 3A, 4A, 5A and 13X. Among these supports, HZSM-5 is preferable.

The catalyst is prepared according to the following process. A solution is prepared by dissolving metal compound precursors such as the chloride, bromide or nitrate of Zn or Mg in deionized water. The molecular sieve is added into the solution and soaked for a period of time under the room temperature. Then the mixture is dried and then calcined to obtain the corresponding catalyst.

In the catalyst, the mass content of Zn is in the range of 0.10% to 18.0%, preferably 0.50% to 15.0%, and more preferably 1.0% to 8.0%. In the catalyst, the mass content of Mg is in the range of 0.10% to 20.0%, preferably 0.50% to 18.0%, and more preferably 1.0% to 15.0%.

In the process, the reaction temperature of alkyl bromides conversion to higher hydrocarbons ( >C₃) is generally in the range of 200°C to 450°C, preferably 220°C to 400°C, and more preferably 240°C to 360°C.

### EXAMPLES

### The preparation of bromomethane from methane

### Example 1

### Catalyst preparation:

Oxalic acid solution (solution A) was prepared by dissolving 6.30 g solid oxalic acid in 100 mL deionized water. 0.10224 g RhCl₃.nH₂O (corresponding 0.40wt% of Rh metal) was dissolved in 50 mL deionized water to obtain solution B. Si(OC₂H₅)₄ (34.581 g) was added into the solution A to obtain a solution containing two phase repelled each other. The solution turned to a clear homogeneous solution after stirred for 1 h in the sealed vessel. The solution B was added to this solution and the mixture was stirred for another 0.5 h, and then dried at 120°C to form a solid. The solid sample was heated from room temperature to 900°C in a period of 4h, calcined at 900°C for 10 h, and cooled down to room temperature in ambient condition, finally crushed and sieved to particles between 20 and 60 mesh to obtain the catalyst 0.40%Rh/SiO₂-900-10. (0.40% refers to the mass ratio of Rh in the catalyst, while -900-10 refers to calcination at 900 °C for 10 h).

### Catalyst evaluation:

The OBM reaction was carried out in a fixed bed reactor (a quartz-tube reactor, i.d. 14 mm) packed with 5.0 g of Rh/SiO₂ catalyst. The gaseous reactants included 20.0 mL/min of CH₄, 5.0 mL/min of O₂ and 5.0 mL/min of N₂ used as an internal standard. The catalyst was contacted with the gaseous reactants for 0.5 hour and then heated from room temperature up to 660°. During the period of heating, HBr/H₂O (6.5 mL/h) was fed into the reactor when the temperature of catalyst bed reached 40°. After 2 hours of steady-going reaction, the resultant gases were sampled for analyzing, and the measurement data were recorded.

The results showed that, the products formed in the reaction include bromomethane, also small amount of dibromomethane, carbon monoxide, carbon dioxide, and trace amount of tribromomethane that can be detected by mass spectrum but not by chromatogram. In the reaction, a methane single-pass conversion of 35.8% with the selectivity for bromomethane of 90.8%, dibromomethane of 2.1%, carbon monoxide of 5.8% and carbon dioxide of 1.3% was obtained.

Similar to Rh/SiO₂ catalyst, the catalyst prepared by chloride, bromide or nitrate of metal selected from the group consisting of Ru, Pd, Pt, Ni, Cu, Zn, Ag, Ce, V, W, Cd, Mo, Mn, Cr and La with SiCl₄, silicon ester or commercial silica sol also showed good catalytic performance. The experiment results are listed in Example 2 to 59.

### Example 2-6

### Catalyst preparation:

According to the method of preparing the catalyst in example 1, solution B was obtained by dissolving corresponding mass of RhCl₃•nH₂O in deionized water, ultimately to give the catalysts of 0.10%Rh/SiO₂-900-10 (example 2), 0.20%Rh/SiO₂-900-10 (example 3), 0.30%Rh/SiO₂-900-10(example 4), 0.50%Rh/SiO₂-900-10 (example 5) and 0.60%Rh/SiO₂-900-10 (example 6).

### Catalyst evaluation:

According to the method of catalyst evaluation in example 1, the following reactions were conducted in a uniform condition. The experimental results were listed in Table 1.

**Table 1. Experimental results of example 1 to 6 (The influences of the mass content of Rh)**

| Examples | Catalysts | Conversion of CH₄(%) | Selectivity(%) | | | |
|---|---|---|---|---|---|---|
| | | | CH₃Br | CH₂Br₂ | CO | CO₂ |
| 2 | 0.10%Rh/SiO₂-900-10 | 31.5 | 87.5 | 5.1 | 6.6 | 0.8 |
| 3 | 0.20%Rh/SiO₂-900-10 | 34.8 | 77.0 | 2.9 | 15.9 | 4.2 |
| 4 | 0.30%Rh/SiO₂-900-10 | 35.3 | 79.6 | 8.5 | 10.2 | 1.7 |
| 1 | 0.40%Rh/SiO₂-900-10 | 35.8 | 90.8 | 2.1 | 5.8 | 1.3 |
| 5 | 0.50%Rh/SiO₂-900-10 | 36.5 | 80.6 | 4.8 | 12.2 | 2.4 |
| 6 | 0.60%Rh/SiO₂-900-10 | 32.0 | 78.3 | 3.1 | 16.3 | 2.3 |

### Example 7-12

### Catalyst preparation:

According to the preparation method of the catalyst in example 1, with just different calcining temperatures and times ,to obtain the following catalysts with different surface areas: 0.40%Rh/SiO₂-900-6(example7), 0.40%Rh/SiO₂-900-2(example8), 0.40%Rh/SiO₂-800-6 (example9), 0.40%Rh/SiO₂-800-2(example10), 0.40%Rh/SiO₂-700-10 (example 11), 0.40%Rh/SiO₂-700-6(example 12).

### Catalyst evaluation:

Catalyst testing was carried out according to the evaluation method of catalyst in example 1, and the results were given in Table 2.

**Table 2. Experimental results of example 1 and 7-12 (The influences of the calcining temperature and time, 0.40%Rh/SiO₂)**

| Example | Calcining temperature(°C)/ time(hr) | Surface area (m²/g) | Conversion of CH₄(%) | Selectivity (%) | | | |
|---|---|---|---|---|---|---|---|
| | | | | CH₃Br | CH₂Br₂ | CO | CO₂ |
| 1 | 900/10 | 0.019 | 35.8 | 90.8 | 2.1 | 5.8 | 1.3 |
| 7 | 900/6 | 0.174 | 36.3 | 82.5 | 2.5 | 12.7 | 2.3 |
| 8 | 900/2 | 0.224 | 36.1 | 80.7 | 1.3 | 15.8 | 2.2 |
| 9 | 800/6 | 4.048 | 35.6 | 84.2 | 2.5 | 11.4 | 1.9 |
| 10 | 800/2 | 17.806 | 36.9 | 72.2 | 0.2 | 22.4 | 5.2 |
| 11 | 700/10 | 267.46 | 25.8 | 22 | 0 | 68.9 | 9.1 |
| 12 | 700/6 | 321.58 | 20.8 | 18 | 0 | 74.2 | 07.8 |

### Example 13-16

### Catalyst preparation:

Catalysts were prepared according to the method of example 1.

### Catalyst evaluation:

Catalyst testing was carried out with just using different reaction temperature according to the evaluation method of catalyst in example 1, and the results were given in Table 3. (600° for example 13, 620° for example 14, 640° for example 15, and 680° for example 16)..

**Table 3. Experimental results of example 1 and 13~16 (The influences of the reaction temperature)**

| example | reaction temperature (°C) | Conversion of C_{H}4(%) | Selectivity (%) | | | |
|---|---|---|---|---|---|---|
| | | | CH₃Br | CH₂Br₂ | CO | CO₂ |
| 13 | 600 | 18.1 | 92.8 | 7 | 0 | 0.3 |
| 14 | 620 | 25.5 | 93.4 | 3.6 | 2.5 | 0.5 |
| 15 | 640 | 31.2 | 90.7 | 3.3 | 4.4 | 1.6 |
| 1 | 660 | 35.8 | 90.8 | 2.1 | 5.8 | 1.3 |
| 16 | 680 | 32.3 | 78.2 | 2.2 | 13.8 | 5.7 |

### Example 17-22

### Catalyst preparation:

Catalysts were prepared according to the method of example 1.

### Catalyst evaluation:

Catalyst testing was carried out with just different HBr/H₂O flow rates according to the evaluation method of catalyst in example 1, and the results were given in Table 4. (3.0 mL/hr for example 17, 4.0 mL/hr for example 18, 5.0 mL/hr for example 19, 6.0 mL/hr for example 20, 7.0 mL/hr for example 21, and 8.0 mL/hr for example 22)

**Table 4. Experimental results of example 1 and 17-22**

| example | HBr/H₂O flow rate (mL/hr) | Conversion of C_{H}4(%) | Selectivity (%) | | | |
|---|---|---|---|---|---|---|
| | | | CH₃Br | CH₂Br₂ | CO | CO₂ |
| 17 | 3 | 27.9 | 55.7 | 2 | 24.7 | 17.6 |
| 18 | 4 | 29.7 | 65.6 | 1.6 | 19.7 | 13 |
| 19 | 5 | 33 | 71 | 1.7 | 18.5 | 8.8 |
| 20 | 6 | 37.4 | 81 | 1.9 | 12.1 | 5 |
| 1 | 6.5 | 35.8 | 90.8 | 2.1 | 5.8 | 1.3 |
| 21 | 7 | 34.7 | 87.8 | 3.1 | 5.9 | 3.2 |
| 22 | 8 | 31.6 | 90 | 4.1 | 3.8 | 2.1 |

### Example 23-25

### Catalyst preparation:

Catalysts were prepared according to the method of example 1.

### Catalyst evaluation:

Catalyst testing was carried out using 10.0 g catalysts according to the evaluation method of catalyst in example 1 (the flow rates of CH₄, O₂, N₂ were 40.0 mL/min, 10.0 mL/min, 0.0 mL/min, respectively), and the results were given in Table 5. (the flow rates of HBr/H₂O were 13.0 mL/hr for example 23, 12.0 mL/hr for example 24 and 10.0 mL/hr for example 25).

**Table 5. Experimental results of example 23-25 (The influences of the different HBr/H₂O flow rate when 10.0 g catalysts were used)**

| example | HBr/H₂O flow rate (mL/hr) | Conversion of C_{H}4(%) | Selectivity (%) | | | |
|---|---|---|---|---|---|---|
| | | | CH₃Br | CH₂Br₂ | CO | CO₂ |
| 23 | 13 | 32.6 | 91.6 | 4.4 | 3.1 | 0.9 |
| 24 | 12 | 33.3 | 91.2 | 3.8 | 4 | 1.1 |
| 25 | 10 | 35.3 | 89.7 | 4.3 | 3.1 | 2.9 |

### Example 26-28

### Catalyst preparation:

Catalysts were prepared according to the method of example 1.

### Catalyst evaluation:

Catalyst testing was carried out using 10.0 g catalysts according to the evaluation method of catalyst in example 1, the flow rates of CH₄, N₂ and HBr/H₂O were 40.0 mL/min, 10.0 mL/min and 10.0 mL/hr, respectively. The flow rates of O₂ were 9.0 mL/min for example 26, 8.0 mL/min for example 27 and 7.0 mL/min for example 28. The results were given in Table 6.

**Table 6. Experimental results of example 25-28.**

| example | O₂ flow rate (mL/min) | Conversion of C_{H}4(%) | Selectivity (%) | | | |
|---|---|---|---|---|---|---|
| | | | CH₃Br | CH₂Br₂ | CO | CO₂ |
| 25 | 10 | 35.3 | 89.7 | 4.3 | 3.1 | 2.9 |
| 26 | 9 | 33.5 | 88.0 | 7.1 | 3.3 | 1.6 |
| 27 | 8 | 30.3 | 88.6 | 7.9 | 2.3 | 1.2 |
| 28 | 7 | 25.8 | 93.3 | 3.6 | 1.9 | 1.2 |

### Example 29-39

### Catalyst preparation:

Catalysts were prepared according to the method of example 1. When preparing these catalysts other metal nitrates (2.0wt%) were added into the RhCl₃•nH₂O solution to get solution B, and then prepared the following catalysts: 2.0%Cu0.40%Rh/SiO₂-900-10 (example 29), 2.0%Zn0.40%Rh/SiO₂-900-10 (example 30), 2.0%Ag0.40%Rh/SiO₂-900-10 (example 3), 2.0%Ce0.40%Rh/SiO₂-900-10 (example 32), 2.0%V0.40%Rh/SiO₂-900-10 (example 33), 2.0%W0.40%Rh/SiO₂-900-10 (example 34), 2.0%Cd0.40%Rh/SiO₂-900-10 (example 35), 2.0%Mo0.40%Rh/SiO₂-900-10 (example 36), 2.0%Mn0.40%Rh/SiO₂-900-10 (example 37), 2.0%Cr0.40%Rh/SiO₂-900-10 (example 38), 2.0%La0.40%Rh/SiO₂-900-10 (example 39), 2.5%Ni0.40%Rh/SiO₂-900-10 (example 40).

### Catalyst evaluation:

Catalyst testing was carried out under the same condition according to the evaluation method of catalyst in example 1, and the results were given in Table 7.

**Table 7. Experimental results of example 1 and 29-39 (The influences of the metal dopants)**

| Example | catalysts | Conversion of CH₄(%) | Selectivity (%) | | | |
|---|---|---|---|---|---|---|
| | | | CH₃Br | CH₂Br₂ | CO | CO₂ |
| 1 | 0.40%Rh/SiO₂-900-10 | 35.8 | 90.8 | 2.1 | 5.8 | 1.3 |
| 29 | 2.0%Cu0.40%Rh/SiO₂-900-10 | 33.9 | 77.1 | 1.7 | 17.8 | 3.4 |
| 30 | 2.0%Zn0.40%Rh/SiO₂-900-10 | 27.7 | 19.4 | 1.3 | 73.7 | 5.5 |
| 31 | 2.0%Ag0.40%Rh/SiO₂-900-10 | 24.4 | 31.3 | 0.4 | 63.1 | 5.2 |
| 32 | 2.0%Ce0.40%Rh/SiO₂-900-10 | 35.3 | 79.8 | 1.8 | 12.6 | 5.8 |
| 33 | 2.0%V0.40%Rh/SiO₂-900-10 | 23.3 | 13 | 0 | 77.5 | 9.5 |
| 34 | 2.0%W0.40%Rh/SiO₂-900-10 | 28.8 | 43.8 | 0.2 | 43 | 13 |
| 35 | 2.0%Cd0.40%Rh/SiO₂-900-10 | 31.4 | 0 | 0 | 86.9 | 13.1 |
| 36 | 2.0%Mo0.40%Rh/SiO₂-900-1 0 | 29.7 | 23.2 | 0 | 66.1 | 10.7 |
| 37 | 2.0%Mn0.40%Rh/SiO₂-900-1 0 | 24.5 | 92.4 | 4.4 | 2.6 | 0.6 |
| 38 | 2.0%Cr0.40%Rh/SiO₂-900-10 | 30.8 | 53.2 | 0.9 | 34 | 11.9 |
| 39 | 2.0%La0.40%Rh/SiO₂-900-10 | 29.1 | 8.9 | 0.7 | 83.6 | 6.8 |
| 40 | 2.5%Ni0.40%Rh/SiO2-900-10 | 29.6 | 84 | 8.8 | 6.2 | 1.0 |

### Example 41-46

### Catalyst preparation:

Catalyst (0.40%Rh/SiO₂-700-10) was prepared according to the method of example 11.

### Catalyst evaluation:

Catalyst testing was carried out under the same condition according to the evaluation method of catalyst in example 1, the flow rates of CH₄, O₂, N₂ were 20.0 mL/min, 10.0 mL/min, 5.0 mL/min, and the flow rate of HBr/H₂O was 8.0 mL/hr. The results were given in Table 8. (The reaction temperature was 560 □ for example 40, 580° for example 41, 600° for example 42, 620° for example 43, 640° for example 44, 660° for example 45)

**Table 8. Experimental results of example 41-46 ( The influences of the reaction temperature)**

| example | Reaction temperature (□) | Conversion of C_{H}4(%) | Selectivity (%) | | | |
|---|---|---|---|---|---|---|
| | | | CH₃Br | CH₂Br₂ | CO | CO₂ |
| 41 | 560 | 45.6 | 61.2 | 4.3 | 27.9 | 6.6 |
| 42 | 580 | 50.5 | 54.9 | 1.2 | 35.9 | 7.9 |
| 43 | 600 | 53.8 | 56.1 | 1.3 | 35.4 | 7.1 |
| 44 | 620 | 54.1 | 55.3 | 2.5 | 35.5 | 6.7 |
| 45 | 640 | 51.6 | 45.2 | 2.1 | 43.5 | 9.2 |
| 46 | 660 | 48.5 | 43.3 | 1.2 | 44.9 | 10.6 |

### Example 47-56

### Catalyst preparation:

Oxalic acid (6.300 g) was dissolved in 100 mL deionized water to get solution A. A certain amount of RuCl₃•nH₂O was dissolved in 50 mL of deionized water to get solution B, adding 34.583 g tetraethoxy-silicone into the solution A to form two phases solution. Stirring the two phases solution under sealing for 1 h to get transparent single phase solution, adding solution B into this solution and stirring for another 0.5 h. The result mixture was dried at 120° and then heated up to 900° at a rate of 200°/hr, maintaining for 10 h and decreased down to ambient temperature, finally sieved into 20-60 mesh to give the following catalysts: 0.10%Ru/SiO2-900-10 (example 46), 0.20%Ru/SiO₂-900-10 (example 47), 0.30%Ru/SiO₂-900-10 (example 48), 0.40%Ru/SiO₂-900-10 (example 49), 0.50%Ru/SiO₂-900-10 (example 50), 0.60%Ru/SiO₂-900-10 (example 51), 0.80%Ru/SiO₂-900-10 (example 52), 1.0%Ru/SiO2₋900-10 (example 53), 1.20%Ru/SiO2₋900-10 (example 54), 1.40%Ru/SiO2-900-10 (example 55).

### Catalyst evaluation:

Catalyst testing was carried out on a fixed bed reaction under normal pressure. The reactor is a quartz tube (inner diameter 14 mm) and 5.00 g catalyst was used. The flow rates of CH₄, O₂ and N₂ were 20.0 mL/min, 5.0 mL/min and 5.0 mL/min, respectively. The concentration of HBr/H₂O is more than 40wt%. The reaction gas was introduced into the reactor for 0.5 h and then the catalyst bed was heated up to 660°, HBr/H₂O solution was directed into the reactor at a rate of 6.5 mL/h when the temperature reached to 400°. After 2h steady reaction, the outlet gas was analyzed and recording the results.

### Example 57

### Preparation of catalyst:

Oxalic acid (6.300 g) was dissolved in 100 mL deionized water to get solution A, a certain amount of RuCl₃•nH₂O and 0.1%Pt salt were dissolved in 50 mL deionized water to get solution B, adding 34.6872 g tetraethoxy-silicone into the solution A to form two phases solution. Stirring the two phases solution under sealing for 1 h to get transparent single phase solution, adding solution B into this solution and stirring for another 0.5 h. The result mixture was dried at 120° and then heated up to 900° at a rate of 200°/hr, maintaining for 10h and decreased down to ambient temperature, finally sieved into 20-60 mesh to give catalyst 0.10%Pt/SiO₂-900-10 (example 57).

### Catalyst evaluation:

Catalyst testing was carried out on a fixed bed reaction under normal pressure. The reactor is a quartz tube (inner diameter 14mm) and 5.00 g catalyst was used. The flow rates of CH₄, O₂ and N₂ were 20.0 mL/min, 5.0 mL/min and 5.0 mL/min, respectively. The concentration of HBr/H₂O is more than 40wt%. The reaction gas was introduced into the reactor for 0.5 h and then the catalyst bed was heated up to 660°, HBr/H₂O solution was directed into the reactor at a rate of 6.5 mL/h when the temperature reached to 400°. After 2h steady reaction, the outlet gas was analyzed and recording the results.

### Example 58-59

### Catalyst preparation:

Oxalic acid (6.300 g) was dissolved in 100 mL deionized water to get solution A, adding 34.5831 g tetraethoxy-silicone into the solution A to form two phases solution. Stirring the two phases' solution in a sealed vessel for 1 h to get transparent single phase solution. The result mixture was dried at 120° and then heated up to 1000° at a rate of 200°/hr, maintaining for 5 h and decreased down to ambient temperature, finally sieved into 20-60 mesh to give support SiO₂.

A certain amount of chlorides of Pd or Pt was dissolved in 50 mL deionized water to get solution B, adding as prepared support SiO₂ into the solution B and stirring for 0.5 h. Then standing 3 h and drying at 120°, finally maintaining 8h at 450° to get catalyst 0.10%Pd/SiO₂-1000-5 (example 58).

### Catalyst evaluation:

Catalyst testing was carried out on a fixed bed reaction under normal pressure. The reactor is a quartz tube (inner diameter 14mm) and 5.00 g catalyst was used. The flow rates of CH₄, O₂ were 20.0 mL/min and 5.0 mL/min, respectively. The concentration of HBr/H₂O is more than 40wt%. The reaction gas was introduced into the reactor for 0.5 h and then the catalyst bed was heated up to 660°, HBr/H₂O solution was directed into the reactor at a rate of 6.0 mL/h when the temperature reached to 400°. After 2 h steady reaction, the outlet gas was analyzed and recording the results. The results were given in Table 9.

**Table 9. Experimental results of example 47-59 (The influences of Ru, Pt and Pd)**

| example | Catalysts | Conversion of CH₄(%) | Selectivity (%) | | | |
|---|---|---|---|---|---|---|
| | | | CH₃Br | CH₂Br₂ | CO | CO₂ |
| 47 | 0.10%Ru/SiO₂-900-10 | 7.2 | 95.5 | 2.6 | 0 | 1.9 |
| 48 | 0.20%Ru/SiO₂-900-10 | 5.9 | 97.0 | 1.8 | 0 | 1.2 |
| 49 | 0.30%Ru/SiO₂-900-10 | 11.6 | 95.5 | 3.8 | 0 | 0.7 |
| 50 | 0.40%Ru/SiO₂-900-10 | 18.4 | 96.5 | 2.8 | 0 | 0.6 |
| 51 | 0.50%Ru/SiO₂-900-10 | 20.2 | 93.1 | 4.6 | 1.3 | 0.9 |
| 52 | 0.60%Ru/SiO₂-900-10 | 23.4 | 88.6 | 4.5 | 3.9 | 3 |
| 53 | 0.80%Ru/SiO₂-900-10 | 25.3 | 91.1 | 5.8 | 2.1 | 1 |
| 54 | 1.0%Ru/SiO₂-900-10 | 32.7 | 84.4 | 4.6 | 6.5 | 4.5 |
| 55 | 1.2%Ru/SiO₂-900-10 | 25.4 | 88.8 | 5.1 | 5.3 | 0.8 |
| 56 | 1.4%Ru/SiO₂-900-10 | 22.0 | 95.6 | 1.1 | 1.9 | 1.4 |
| 57 | 0.10%Pt/SiO₂-900-10 | 7 | 93 | 0 | 7 | 0 |
| 58 | 0.10%Pd/SiO₂-1000-5 | 21 | 15.7 | 0.1 | 67.2 | 17 |
| 59 | 0.10%Pt/SiO₂-1000-5 | 11.9 | 37.7 | 0.2 | 58.1 | 4 |

### The preparation of hydrocarbons from bromomethane

### Example 60

### Preparation of catalyst:

Zn(NO₃)₂• 6H₂O (2.8856 g) was added into 100 mL deionized water to get a clear solution, adding HZSM-5 (15.00 g) into the solution, stirring under sealing for 40-60 min, impregnating for 12 h, reacting at 90° water bath for 4 h, drying at 120° and then maintaining at 450° for 8 h, finally sieved into 20-60 mesh to give catalyst 5.0%Zn/HZSM-5-450-8.

### Catalyst evaluation:

Catalyst testing was carried out on a fixed bed reaction under normal pressure. The reactor is a quartz tube (inner diameter 14mm) and 8.00 g catalyst was used. The carrier gas is N₂ and the flow rates of CH₃Br was 7.76 mL/min. The carrier gas was introduced into the reactor for 0.5h and then the catalyst bed was heated up to 300°. After 2 h steady reaction, the outlet gas was analyzed and recording the results.

The results were: the conversion of bromomethane was 99.63%, gas products were alkanes, alkenes between C₂-C₅ and residual bromomethane, liquid products were alkanes, alkenes and aromatics between C₅-C₁₃.

### Example 61-65

### Preparation of catalyst:

Catalyst (5.0%Zn/HZSM-5-450-8) was prepared according to the method of example 59.

### Catalyst evaluation:

Catalyst testing was carried out just using different reaction temperature according to the evaluation method of catalyst in example 59, 280° for example 61, 260° for example 62, 240° for example 63, 220° for example 64, 200° for example 65, and the results were given in talbe 10.

**Table 10. Experimental results of example 60-65 (The influences of reaction temperature).**

| example | Reaction temperature (°C) | Conversion of CH₃Br (%) |
|---|---|---|
| 60 | 300 | 99.63 |
| 61 | 280 | 99.51 |
| 62 | 260 | 99.10 |
| 63 | 240 | 40.56 |
| 64 | 220 | 24.08 |
| 65 | 200 | 21.74 |

### Example 66-71

### Catalyst preparation:

According to the preparation method of example 59, different amounts of Zn(NO₃)₂•6H₂O were used to obtain the following catalysts: 1.0%Zn/HZSM-5-450-8 (example 66), 3.0% Zn/HZSM-5-450-8 (example 67), 8.0%Zn/HZSM-5-450-8 (example 68), 10.0%Zn/HZSM-5-450-8 (example 69), 12.0%Zn/HZSM-5-450-8 (example 70), 15.0%Zn/HZSM-5-450-8 (example 71).

### Catalyst evaluation:

Catalyst testing was carried out according to the method of example 59. The catalysts were activated at 300° for 4h before reaction at 260°. The results were given in table 11.

**Table 11. Experimental results of example 62 and 65-70 (The influences of ZnO content).**

| example | catalysts | Conversion of CH₃Br (%) |
|---|---|---|
| 66 | 1.0%Zn/HZSM-5-450-8 | 99.30 |
| 67 | 3.0% Zn/HZSM-5-450-8 | 99.71 |
| 62 | 5.0%Zn/HZSM-5-450-8 | 99.10 |
| 68 | 8.0%Zn/HZSM-5-450-8 | 99.28 |
| 69 | 10.0%Zn/HZSM-5-450-8 | 97.24 |
| 70 | 12.0%Zn/HZSM-5-450-8 | 80.29 |
| 71 | 15.0%Zn/HZSM-5-450-8 | 80.97 |

### Example 72-77

### Catalyst preparation:

According to the preparation method of example 59, different zeolite supports(HY, H, 3A, 4A, 5A, 13X) were used to give the following catalysts: 3.0% Zn/HY-450-8(example 72), 3.0% Zn/Hβ-450-8 (example 73), 3.0% Zn/3A-450-8(example 74), 3.0% Zn/4A-450-8(example 75), 3.0% Zn/5A-450-8(example 76), 3.0% Zn/13X-450-8(example 77).

### Catalyst evaluation:

Catalyst testing was carried out according to the method of example 59. The catalysts were activated at 300° for 4h before reaction at 260°. The results were given in Table 12.

**Table 12. Experimental results of example 72-77 (The influences of supports)**

| example | catalysts | Conversion of CH₃Br (%) |
|---|---|---|
| 72 | 3.0% Zn/ HY-450-8 | 46.65 |
| 73 | 3.0%Zn/ Hβ-450-8 | 46.64 |
| 74 | 3.0%Zn/3A-450-8 | 0 |
| 75 | 3.0%Zn/4A-450-8 | 30.50 |
| 76 | 3.0%Zn/5A-450-8 | 15.98 |
| 77 | 3.0%Zn/13X-450-8 | 22.81 |

### Example 78-84

### Catalyst preparation:

Mg(NO₃)₂• 6H₂O (corresponding percentage content) were dissolved into 100 mL deionized water and stirred to get clear solution, adding HZSM-5(Si/A1=360, 15.00 g) into the solution, stirring under sealing for 40-60 min and standing for 12 h. After reacting at 90° water bath for 4 h, the solution was dried at 120° and calcined at 450° for 8h. Finally sieved into 20-60 mesh to give the following catalysts: (1.0%Mg/HZSM-5-450-8(example 78), 3.0%Mg/HZSM-5-450-8(example 79), 5.0%Mg/HZSM-5-450-8(example 80), 8.0%Mg/HZSM-5-450-8(example 81), 10.0%Mg/HZSM-5-450-8(example 82), 15.0%Mg/HZSM-5-450-8(example 83), 18.0%Mg/HZSM-5-450-8(example 84).

### Catalyst evaluation:

Catalyst testing was carried out according to the method of example 59. The catalysts were activated at 300° for 4 h before reaction at 260°. The results were given in Table 13.

**Table 13. Experimental results of example 78-84 (The influences of MgO content)**

| example | catalysts | Conversion of CH₃Br (%) |
|---|---|---|
| 78 | 1.0%Mg/HZSM-5-450-8 | 98.74 |
| 79 | 3.0%Mg/HZSM-5-450-8 | 99.74 |
| 80 | 5.0%Mg/HZSM-5-450-8 | 99.60 |
| 81 | 8.0%Mg/HZSM-5-450-8 | 99.59 |
| 82 | 10.0%Mg/HZSM-5-450-8 | 99.75 |
| 83 | 15.0%Mg/HZSM-5-450-8 | 99.50 |
| 84 | 18.0%Mg/HZSM-5-450-8 | 75.50 |

## Claims

1. A catalyst used for the oxidative bromination reaction of methane, wherein the catalyst is a compound catalyst of metals or metal oxides distributing in silica, prepared by the hydrolyzation, drying, and then calcining of the mixture formed by corresponding soluble metal compound precursors and silicon precursors; wherein the metal compound precursor comprises at least one soluble chloride, bromide or nitrate of metal selected from the group consisting of Ru, Rh, Pd, Pt, Ni, Cu, Zn, Ag, Ce, V, W, Cd, Mo, Mn, Cr and La, and the silicon precursor is selected from SiCl₄, silicon ester or silica sol solution.

2. The catalyst of claim 1, the said metal compound precursor comprises chloride, bromide or nitrate of Ru or Rh.

3. The catalyst of claim 2, wherein the mass content of Ru in the catalyst is in the range of 0.10% to 2.0%.

4. The catalyst of claim 3, wherein the mass content of Ru in the catalyst is in the range of 0.40% to 1.40%.

5. The catalyst of claim 4, wherein the mass content of Ru in the catalyst is in the range of 0.60% to 1.20%.

6. The catalyst of claim 2, wherein the mass content of Rh in the catalyst is in the range of 0.10% to 0.80%.

7. The catalyst of claim 6, wherein the mass content of Rh in the catalyst is in the range of 0.20% to 0.50%.

8. The catalyst of claim 7, wherein the mass content of Rh in the catalyst is in the range of 0.30% to 0.50%.

9. The catalyst of claim 1, wherein the temperature of calcining is in the range of 500° to 1200°.

10. The catalyst of claim 9, wherein the temperature of calcining is in the range of 700° to 1000°.

11. The catalyst of claim 10, wherein the temperature of calcining is in the range of 700° to 900°.

12. The catalyst of claim 1, wherein the reaction temperature is in the range of 500° to 750°.

13. The catalyst of claim 12, wherein the reaction temperature is in the range of 600° to 800°.

14. The catalyst of claim 13, wherein the reaction temperature is in the range of 620° to 670°.

15. A catalyst used for the alkyl bromides conversion to C₃ to Cl₃ hydrocarbons, wherein the catalyst is prepared by impregnating the support in the solution of active metal compound precursors, and subsequently drying and calcining the solution, wherein the said metal compound precursors comprises chloride, bromide or nitrate of Zn or Mg.

16. The catalyst of claim 15, wherein the molecular sieve support is selected from the group of HZSM-5, HY, Hβ, 3A, 4A, 5A and 13X.

17. The catalyst of claim 16, wherein the support is HZSM-5.

18. The catalyst of claim 15, wherein the mass content of Zn in the catalyst is in the range of 0.10% to 18.0%

19. The catalyst of claim 18, wherein the mass content of Zn in the catalyst is in the range of 0.50% to 15.0%.

20. The catalyst of claim 19, wherein the mass content of Zn in the catalyst is in the range of 1.0% to 8.0%.

21. The catalyst of claim 15, wherein the mass content of Mg in the catalyst is in the range of 00.10% to 20.0%.

22. The catalyst of claim 21, wherein the mass content of Mg in the catalyst is in the range of 0.50% to 18.0%.

23. The catalyst of claim 22, wherein the mass content of Mg in the catalyst is in the range of 1.0% to 15.0%.

24. The catalyst of claim 15, wherein the reaction temperature is in the range of 200° to 450°.

25. The catalyst of claim 24, wherein the reaction temperature is in the range of 220° to 400°.

26. The catalyst of claim 25, wherein the reaction temperature is in the range of 240° to 360°.
